# EUROPÄISCHE PATENTSCHRIFT

(11) **EP 0 588 225 B1**
(45) Veröffentlichungstag und Bekanntmachung des Hinweises auf die Patenterteilung: **18.06.1997**
(21) Anmeldenummer: 93114394.5
(22) Anmeldetag: 08.09.1993
(51) Int. Cl.: C07C 45/50, C07C 47/02, C07C 45/80

(54) **Verfahren zur Herstellung von Aldehyden**
Process for the preparation of aldehydes
Procédé pour la préparation d'aldéhydes

(30) Priorität: 16.09.1992 DE 4230871; 14.05.1993 DE 4316180
(43) Veröffentlichungstag der Anmeldung: 23.03.1994
(73) Patentinhaber: BASF Aktiengesellschaft, 67063 Ludwigshafen (DE)
(72) Erfinder: Kneuper, Heinz-Josef, Dr., D-6800 Mannheim 1 (DE); Roeper, Michael, Prof. Dr., D-6706 Wachenheim (DE); Paciello, Rocco, Dr., D-6702 Bad Duerkheim (DE)

(56) Entgegenhaltungen:
- GB-A- 1 296 435
- US-A- 4 292 196
- US-A- 4 298 499

## Beschreibung

Die vorliegende Erfindung betrifft ein Verfahren zur Herstellung von Aldehyden durch die Hydroformylierung von Olefinen mit mehr als 7 Kohlenstoffatomen, umfassend die Stufe der Hydroformylierung mittels eines homogen im Reaktionsmedium gelösten Rhodium-Katalysators, die Abtrennung des Rhodium-Katalysators vom Austrag der Hydroformylierungsreaktion und die Rückführung des vom Hydroformylierungsaustrag abgetrennten Rhodiums in die Hydroformylierungsstufe.

Die Hydroformylierung von Olefinen mit Kohlenmonoxid und Wasserstoff in Gegenwart von Übergangsmetall-Katalysatoren ist bekannt. Während α-Olefine sehr gut mit rhodiumhaltigen, phosphinmodifizierten Katalysatoren hydroformylierbar sind (vgl. J. Falbe, Ed: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, S. 95 ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes vom homogen im Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

US-A 4 292 196 und US-A 4 298 499 betreffen Verfahren zur Hydroformylierung von Olefinen in Gegenwart eines Carbonylkomplexes eines Metalls der VIII. Nebengruppe und in Gegenwart unter anderem eines Aminliganden, wobei der Katalysator nach beendeter Reaktion in die wäßrige Phase extrahiert wird.

GB-A 1 296 435 betrifft ein Verfahren zur Hydroformylierung von Olefinen mittels homogener Rhodiumkatalysatoren, die mit einem zweizähnigen, stickstoff- und sauerstoff-enthaltenden Liganden; z. B. Salicylaldoxim, modifiziert sind.

Hingegen lassen sich interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit phosphorhaltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe, S. 38 ff). Die Begriffe "nacktes Rhodium" oder "nackte Rhodium-Katalysatoren" werden in dieser Anmeldung für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphin- oder Phosphit-Liganden, modifiziert sind. Als Liganden in diesem Sinne werden nicht Carbonyl- oder Hydrido-Liganden verstanden. Es wird in der Fachliteratur (s. Falbe, S. 38 ff) angenommen, daß die Rhodium-Verbindung HRh(CO)₄ die katalytisch aktive Rhodiumspezies bei der Hydroformylierung mit "nackten Rhodium-Katalysatoren" ist, obgleich dies aufgrund der vielen in der Hydroformylierungsreaktionszone nebeneinander ablaufenden Chemismen nicht eindeutig bewiesen ist. Lediglich der Einfachheit halber machen wir auch in dieser Anmeldung von dieser Annahme Gebrauch, ohne daß dadurch eine Einschränkung des Schutzumfangs der vorliegenden Anmeldung in Betracht zu ziehen wäre, falls sich zukünftig einmal eine andere Rhodiumspezies als die genannte, als die eigentlich katalytisch aktive, herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonyl-Verbindungen, wie Rh₄(CO)₁₂ und Rh₆(CO)₁₆ oder aus Organo-Rhodium-Verbindungen, wie Rhodiumdicarbonylacetonylacetonat, Cyclooctadien-Rhodiumacetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgenden Literaturstellen verwiesen: US-A 4 400 547; DE-A 33 38 340; DE-A 26 04 545; WO 82/03856; Chem. Ber. 102, 2238 (1969); Tetrahedron Lett. 29, 3261 (1968); Hydrocarbon Process. 85-86 (1975).

Allerdings hat auch die Hydroformylierung mit "nacktem" Rhodium den Nachteil, daß sich der thermolabile Rhodium-Katalysator (vgl. US-A 4 400 547) infolge der thermischen Belastung bei der destillativen Aufarbeitung des Hydroformylierungsproduktes teilweise zu metallischem Rhodium zersetzt, das sich an den Wandungen des Reaktors und der Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

In DE-A 33 38 340 und US-A 4 400 547 werden Verfahren zur Hydroformylierung mittels "nackter Rhodium-Katalysatoren" beschrieben, bei denen zur Verhinderung der Rhodiumabscheidung dem Reaktionsaustrag der Hydroformylierung ein Phosphin oder Phosphit zugesetzt wird, welche den Rhodium-Katalysator durch die Bildung von Phosphin- und/oder Phosphit-Komplexen vor einer thermischen Zersetzung im Zuge der destillativen Aufarbeitung des Hydroformylierungsaustrags schützen. Nach Abschluß der Destillation wird der rhodiumhaltige Destillationssumpf mit einem Oxidationsmittel behandelt, wobei das Rhodium in katalytisch aktiver Form aus den betreffenden Phosphin- oder Phosphit-Komplexen freigesetzt wird und die Phosphin- und Phosphit-Liganden zu den entsprechenden, unter Hydroformylierungsbedingungen keine Rhodium-Komplexe bildenden Phosphinoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden.

WO 82/03856 betrifft ein Verfahren zur Thermostabilisierung von unmodifizierten, also "nackten Rhodium-Katalysatoren", in dem der Austrag aus der Hydroformylierungsreaktion mit einem sauerstoffhaltigen Gas behandelt wird, wodurch die gebildeten Aldehyde z.T. zu den entsprechenden Carbonsäuren oxidiert werden, welche mit dem Rhodium-Katalysator bei der destillativen Aufarbeitung thermostabile Rhodium-Carboxylate bilden, die wieder als Katalysatoren zur Hydroformylierung verwendet werden können. Nachteilig an diesem Verfahren ist die Verringerung der Ausbeute, als Folge der partiellen Oxidation der Produktaldehyde zu Carbonsäuren. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Zur Vermeidung von Rhodiumverlusten wurde gemäß US-A 3 984 478 ein Hydroformylierungsverfahren entwickelt, bei dem die Hydroformylierung in Gegenwart eines gegebenenfalls sulfonierten Phthalocyanins durchgeführt wird. Da die hierbei gebildeten Rhodium-Phthalocyanin-Komplexe z.T. schwer löslich oder nur in Wasser, nicht jedoch im organischen Hydroformylierungsmedium löslich sind, wird die Hydroformylierung wahlweise in Gegenwart der festen Rhodium-Phthalocyanine oder im Zwei-Phasen-System mit Wasser durchgeführt. Allerdings ist die koordinative Bindung des Rhodiums zum Phthalocyanin in diesen Komplexen sehr fest, so daß das Rhodium auch unter den Hydroformylierungsbedingungen ans Phthalocyanin gebunden bleibt. Als Folge hiervon findet die Hydroformylierungsreaktion nur an der Grenzfläche Hydroformylierungsmedium/festes Phthalocyanin bzw. an der Grenzfläche zur wäßrigen Rhodium-Phthalocyanin-Komplexlösung statt, wodurch die Reaktionsgeschwindigkeit und damit auch die Raum-Zeit-Ausbeute der Hydroformylierungsreaktion so niedrig wird, daß sich dieses Verfahren nicht wirtschaftlich betreiben läßt.

Der vorliegenden Erfindung lag daher die Aufgabe zugrunde, ein Verfahren zur Herstellung von Aldehyden aus langkettigen und/oder verzweigten Olefinen mit Hilfe "nackter Rhodium-Katalysatoren" zu finden, mit dem zum einen die Probleme der Abscheidung metallischen Rhodiums bei der destillativen Aufarbeitung des Hydroformylierungsproduktes sowie der Rhodium-Katalysator-Abtrennung von nicht destillierbaren Produktaldehyden zufriedenstellend gelöst werden können. Es sollte zu diesem Zweck nach einem Hydroformylierungsverfahren gesucht werden, in dem sich Komplex-Liganden reversibel und in Abhängigkeit vom Druck der Kohlenmonoxid-Wasserstoff-Gasmischung an den Rhodium-Katalysator koordinativ binden, so daß dieser bei einer extraktiven Aufarbeitung extrahierbar wird. Nach Rückführung in die Hydroformylierungsreaktion sollten die so gebildeten Komplexe unter dem angewandten Reaktionsdruck in Gegenwart der Kohlenmonoxid-Wasserstoff-Gasmischung reversibel dekomplexieren und die freigesetzte Rhodiumverbindung wieder die katalytischen Eigenschaften des "nackten Rhodiums" annehmen.

Dementsprechend wurde ein Verfahren zur Herstellung von Aldehyden durch die Hydroformylierung von Olefinen mit mehr als 7 Kohlenstoffatomen, umfassend die Stufe der Hydroformylierung mittels eines homogen im Reaktionsmedium gelösten nackten Rhodium-Katalysators, die Abtrennung des nackten Rhodium-Katalysators vom Austrag der Hydroformylierungsreaktion und die Rückführung des vom Hydroformylierungsaustrag abgetrennten Rhodiums in die Hydroformylierungsstufe gefunden, das dadurch gekennzeichnet ist, daß man den nackten Rhodium-Katalysator aus dem Hydroformylierungsaustrag mittels einer wäßrigen Lösung eines stickstoffhaltigen Komplexbildners aus der Gruppe der gegebenenfalls substituierten, sulfonierten oder sulfonierte Substituenten tragenden 2,2'-Bipyridine, 1,10-Phenanthroline, 2,2'-Bichinoline, 2,2',6',2''-Terpyridine und Porphine, und/oder aus der Gruppe der gegebenenfalls substituierten, carboxylierten oder carboxylierte Substituenten tragenden 2,2'-Bipyridine, 1,10-Phenanthroline, 2,2'-Bichinoline, 2,2',6',2''-Terpyridine und Porphine in die wäßrige Phase extrahiert und den wäßrigen, rhodiumhaltigen Extrakt in die Hydroformylierungsstufe zurückführt.

Erfindungsgemäß werden die bei der Hydroformylierung mit "nacktem Rhodium" erhaltenen rhodiumhaltigen Hydroformylierungsausträge mit wasserlöslichen, stickstoffhaltigen Komplexbildnern, vorzugsweise mehrzähnigen Komplexbildnern, versetzt, die mit dem Rhodium-Katalysator Komplexe bilden, welche hydrophil sind und infolge ihrer guten Wasserlöslichkeit mit Wasser aus dem organischem Medium des Hydroformylierungsaustrags extrahiert werden können. Nach der extraktiven Abtrennung des im Hydroformylierungsaustrag enthaltenen Rhodium-Katalysators in Form eines wasserlöslichen Komplexes mit dem erfindungsgemäß eingesetzten Komplexbildner, kann das Hydroformylierungsprodukt auf an sich übliche Weise aufgearbeitet werden, beispielsweise indem man das Hydroformylierungsprodukt aus dem organischen Extrakt destillativ isoliert oder indem man leichter flüchtige organische Bestandteile des Hydroformylierungsaustrags vom schwerer flüchtigen oder gegebenenfalls sogar undestillierbaren Hydroformylierungsprodukt abdestilliert. Der wäßrige Extrakt des Hydroformylierungsaustrags, welcher den nunmehr vom stickstoffhaltigen Komplexbildner komplexierten Rhodium-Katalysator enthält, wird als solcher in den Hydroformylierungsreaktor geleitet. Unter dem dort herrschenden Kohlenmonoxid-Wasserstoff-Druck wird das Rhodium durch das Kohlenmonoxid aus seinem wasserlöslichen Komplex mit dem erfindungsgemäß eingesetzten, stickstoffhaltigen Komplexbildner unter Rückbildung des lipophilen, wasserunlöslichen "nackten Rhodium-Katalysators" herausgelöst, welcher dann aus der wäßrigen Phase in die organische Phase des Hydroformylierungsmediums migriert und dort wieder die Hydroformylierung der eingesetzten Olefine mit der für "nacktes Rhodium" charakteristischen Reaktivität und Selektivität katalysiert.

Als Komplexbildner, die mit dem im Austrag der Hydroformylierungsreaktion gelösten Rhodium-Katalysator wasserlösliche Komplexe bilden werden vorzugsweise mehrzähnige, insbesondere zwei-, drei- oder vierzähnige, sulfonierte stickstoffhaltige Komplexbildner verwendet, besonders bevorzugt sind 2,2'-Bipyridin-Sulfonate, 2,2'-Bichinolin-Sulfonate, 1,10-Phenanthrolin-Sulfonate, 2,2',6',2''-Terpyridin-Sulfonate oder Porphin-Sulfonate. Weiterhin ist die Verwendung mehrzähniger, insbesondere die Verwendung zwei-, drei- oder vierzähniger, carboxylierter, stickstoffhaltiger Komplexbildner im erfindungsgemäßen Verfahren besonders bevorzugt, insbesondere die Verwendung von 2,2'-Bipyridin-Carboxylaten, 1,10-Phenanthrolin-Carboxylaten, 2,2'-Bichinolin-Carboxylaten, 2,2',6',2''-Terpyridin-Carboxylaten und Porphin-Carboxylaten.

Unter Einwirkung dieser Komplexbildner bilden sich vermutlich über das freie Elektronenpaar der Stickstoffatome koordinative Bindungen mit dem Rhodium-Zentralatom des Rhodium-Katalysators aus, wobei vermutlich ein Teil des an das zentrale Rhodiumatom des Rhodium-Katalysators gebundenen Kohlenmonoxids unter den Bedingungen der Komplexbildung durch diese Liganden reversibel verdrängt wird. Die Anwesenheit von Sulfonat- oder Carboxylatgruppen in den erfindungsgemäß verwendeten, stickstoffhaltigen Komplexbildnern ist kritisch für die Durchführbarkeit des erfindungsgemäßen Verfahrens. Die Komplexbildner können eine oder mehrere Carboxylat- und/oder Sulfonat-Gruppen pro Molekül enthalten, wobei die Anzahl der Carboxylat- und/oder Sulfonat-Gruppen im Molekül selbstverständlich auch von der Molekülgröße des Komplexbildners und dessen Reaktivität gegenüber Sulfonierungsreagenzien abhängig ist. Beispielsweise werden in der Regel monosulfonierte 2,2'-Bipyridine als Komplexbildner verwendet, wohingegen die sulfonierten Porphine beispielsweise vier Sulfonat-Gruppen im Molekül enthalten können. Da Carboxylat-Gruppen tragende stickstoffhaltige Komplexbildner vorteilhaft durch die Oxidation der entsprechenden Alkyl-, vorzugsweise Methyl-substituierten Komplexbildner hergestellt werden können, die nach konventionellen chemischen Verfahren leicht zugänglich sind, können die carboxylierten Komplexbildner je nach Molekülgröße 1 bis 6, vorzugsweise 1 bis 4 und besonders bevorzugt 1 bis 3 Carboxylgruppen tragen. Die Anzahl der Carboxyl- und/oder Sulfonsäure-Gruppen im Komplexbildner-Molekül beeinflußt die Wasserlöslichkeit dieser Komplexbildner. Selbstverständlich können im erfindungsgemäßen Verfahren auch stickstoffhaltige Komplexbildner eingesetzt werden, die sowohl Carboxylgruppen als auch Sulfonsäuregruppen als Substituenten enthalten oder Gemische sulfonierter und carboxylgruppenhaltiger Komplexbildner verwendet werden. Die Sulfonat- und Carboxylat-Gruppen liegen in den erfindungsgemäß angewandten Komplex-bildnern vorzugsweise in Salzform vor, insbesondere in Form von wasserlöslichen Salzen, besonders bevorzugt in Form ihrer Onium-, Alkalimetall- und/oder Erdalkalimetallsalze. Die Art des verwendeten Oniumsalzes ist im allgemeinen nicht kritisch. So können z.B. Ammonium-, Phosphonium- oder Arsoniumsalze der betreffenden Carbon- oder Sulfonsäuren eingesetzt werden. Zur Vermeidung von Mißverständnissen sei an dieser Stelle ausdrücklich darauf hingewiesen, daß die erfindungsgemäß verwendbaren stickstoffhaltigen Komplexbildher-Sulfonate oder -Carboxylate die Sulfonat- oder Carboxylatgruppen als Substituenten tragen und nicht etwa salzartig mit irgendwelchen Sulfonat- oder Carboxylatanionen verbunden sind.

Die genannten Komplexbildner können zusätzlich mit unter den Reaktionsbedingungen inerten Substituenten wie Halogenatomen, insbesondere Fluor-, Chlor- oder Bromatomen, Alkylgruppen, insbesondere C₁- bis C₄-Alkylgruppen, Arylgruppen, insbesondere C₆- bis C₁₀-Arylgruppen, C₇- bis C₁₂-Aralkylgruppen, der Nitrogruppe, der Hydroxygruppe, der Cyanogruppe, Alkoxygruppen, insbesondere C₁- bis C₄-Alkoxygruppen sowie C₁- bis C₁₀-Alkylsulfonatgruppen substituiert sein.

Eine Substitution mit Alkyl-, Aryl- oder Aralkylgruppen kann insbesondere dann von Vorteil sein, wenn der stickstoffhaltige Komplexbildher reaktionsträge ist und nur unter drastischen Bedingungen sulfoniert werden kann. Da carbocyclische Aryl- und Aralkylgruppen oftmals leichter zu sulfonieren sind als elektronenarme, stickstoffhaltige Heterocyclen, wie Pyridin oder Bipyridin, läßt sich im Falle einer derartigen Substitution des Heterocyclus oder im Falle einer Anellierung aromatischer, nicht-heterocyclischer Ringsysteme an den stickstoffhaltigen Heterocyclus die Sulfonierung unter wesentlich milderen Bedingungen ausführen, ohne daß diese derart substituierten, stickstoffhaltigen Komplexbildner durch eine solche Substitution in ihrer Eigenschaft als Komplexbildher für das erfindungsgemäße Verfahren beeinträchtigt werden. Ähnliches gilt auch für den Fall einer Alkylsubstitution des stickstoffhaltigen Komplexbildners, da die aliphatischen Seitenketten im allgemeinen leicht z.B. mittels Sulfurylchlorid in die entsprechenden Sulfochloride umgewandelt werden können, deren basische Hydrolyse die entsprechenden Sulfonate ergibt.

Die sulfonierten stickstoffhaltigen Komplexbildner können aus den entsprechenden, nicht sulfonierten Stammverbindungen nach an sich herkömmlichen Sulfonierungsverfahren, wie der Umsetzung mit konzentrierter Schwefelsäure oder Oleum, gegebenenfalls in Gegenwart von Katalysatoren, wie Quecksilbersulfat, oder durch die Umsetzung dieser Verbindungen mit Halogensulfonsäuren, vorzugsweise mit Chlorsulfonsäure und anschließender Hydrolyse der gebildeten Sulfonsäurehalogenide zu den Sulfonsäuresalzen, erzeugt werden. Alkansulfonat - substituierte Komplexbildher, sind z.B. durch die Sulfochlorierung der entsprechenden Stammverbindungen, mittels Sulfurylchlorid und die anschließende alkalische Hydrolyse des gebildeten Sulfochlorids zugänglich. Diese und andere zur Herstellung der sulfonierten Komplexbildher anwendbare Methoden sind in C. Ferri, Reaktionen der organischen Synthese, S. 165 - 172 und S. 484 - 485, Thieme, Stuttgart 1978 beschrieben. Beispielsweise ist 2,2'-Bipyridin-5-Sulfonsäure durch die Sulfonierung von 2,2'-Bipyridin nach dem Verfahren von Herrmann et al., Chem.Ber. 123, 1953 (1990) erhältlich. 1,10-Phenanthrolin kann auf die analoge Weise sulfoniert werden. Weitere mit Sulfonsäuregruppen substituierte Bipyridine können nach Campa et al., An. Quim., Ser. C 84, 128 (1988) hergestellt werden. Die Terpyridine, die z.B. nach den von Kröhnke in Synthesis 1 (1976) angegebenen Methoden hergestellt werden können, lassen sich ebenfalls nach den von Ferri angegebenen Verfahren in die betreffenden Sulfonate überführen.

Bei der Sulfonierung der stickstoffhaltigen Komplexbildner nach den zuvor angegebenen Methoden werden in der Regel Isomerengemische der sulfonierten Komplexbildher gebildet, die die Sulfonatgruppen an den verschiedenen möglichen Positionen des stickstoffhaltigen Komplexbildher-Ringsystems und/oder an den an das Komplexbildher-Ringsystem gebundenen Substituenten, insbesondere an den aromatischen Substituenten, wie den Phenyl- oder Naphthyl-Substituenten, tragen. Außerdem kommt es, insbesondere bei den komplexeren Komplexbildher-Ringsystemen sowie bei den mit aromatischen Substituenten substituierten Komplexbildnern, häufig zur Mehrfachsulfonierung dieser Komplexbildner. Die so nach den verschiedenen Sulfonierungsmethoden erhaltenen Komplexbildnersulfonat-Gemische, bestehend aus isomeren, einfach sulfonierten Komplexbildnern und mehrfach sulfonierten Komplexbildnern, können selbstverständlich nach den Verfahren des Standes der Technik, wie Kristallisation oder Ionenaustauschchromatographie, in die einzelnen sulfonierten Komponenten zerlegt und die so erhaltenen Einzelkomponenten als Komplexbildner im erfindungsgemäßen Verfahren eingesetzt werden, da aber der Ort der Sulfonierung als auch der Sulfonierungsgrad der sulfonierten Komplexbildner für das Ergebnis des erfindungsgemäßen Verfahrens im allgemeinen nicht kritisch sind und es für den Erfolg des erfindungsgemäßen Verfahrens im allgemeinen nur darauf ankommt, daß die stickstoffhaltigen Komplexbildner sulfoniert sind, werden im erfindungsgemäßen Verfahren vorzugsweise die nach den verschiedenen Sulfonierungsmethoden erhaltenen Gemische der Komplexbildnersulfonate, vorteilhaft nach deren Überführung in ihre wasserlöslichen Salze, verwendet, wodurch sich die Auftrennung in die sulfonierten Einzelkomponenten des Komplexbildnersulfonatgemisches erübrigt. Aus diesem Grunde wird für den Zweck dieser Anmeldung in der Regel auch nicht von den individuellen Sulfonaten der verschiedenen Komplexbildner gesprochen, sondern stattdessen die betreffenden Komplexbildner gruppenweise bezeichnet, z.B. als 2,2'-Bichinolin-Sulfonate, 1,10-Phenanthrolin-Sulfonate, Terpyridin-Sulfonate usw. Nur in Ausnahmefällen, wenn bei bestimmten Sulfonierungsverfahren zur Sulfonierung spezieller Komplexbildher der betreffende sulfonierte Komplexbildner unter den üblicherweise angewandten Reaktionsbedingungen praktisch ausschließlich entsteht, wird in dieser Anmeldung namentlich vom individuellen, sulfonierten Komplexbildner gesprochen.

Zur Verdeutlichung der Vielzahl der erfindungsgemäß anwendbaren Komplexbildner werden im folgenden beispielhaft eine Reihe von stickstoffhaltigen Komplexbildnern genannt, die sich nach Sulfonierung, entsprechend den zuvor angegebenen Methoden und Überführung in ihre Salze, im erfindungsgemäßen Verfahren einsetzen lassen: 2,2'-Bipyridin, 2,2'-Bichinolin der Formel I 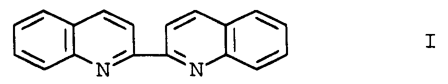 5,6,5',6'-Dibenzo-2,2'-bichinolin der Formel II 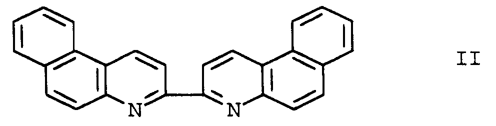 1,10-Phenanthrolin, 2,9-Dimethylphenanthrolin, 4,7-Diphenyl-1,10-phenanthrolin ("Bathophenanthrolin") der Formel III 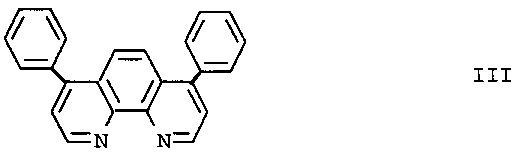 2,9-Dimethyl-4,7-diphenyl-1,10-phenanthrolin ("Bathocuproin") der Formel IV 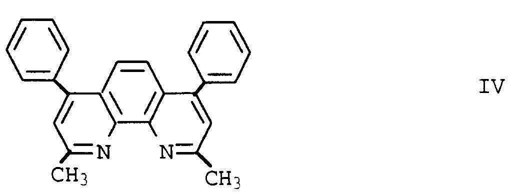 4,5-Diazafluoren V 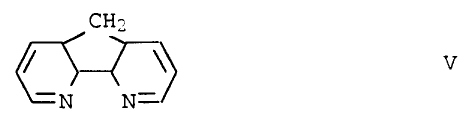 Dipyrido[3,2-a: 2',3'-c]phenazin der Formel VI (Verbindungen V und VI sind erhältlich nach Aust. J. Chem. 23, 1023 (1970)) 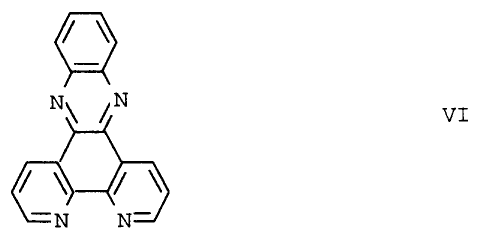 2,2',6',2''-Terpyridin der Formel VII 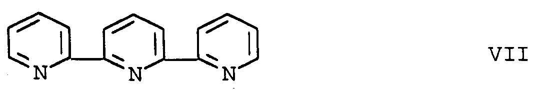 4'-Phenyl-2,2',6',2''-Terpyridin der Formel VIII 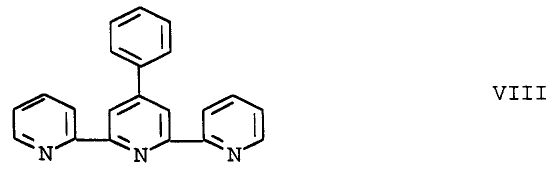 4-Methyl-(4'-phenyl)-(4''-methyl)-2,2',6',2''-terpyridin der Formel IX 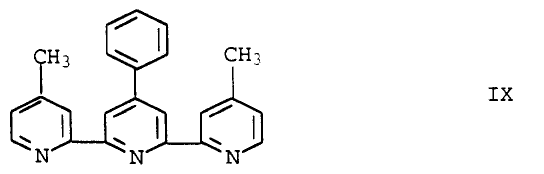 sowie die Porphine.

Als besonders gut geeignet für das erfindungsgemäße Verfahren haben sich die sulfonierten stickstoffhaltigen Komplexbildner erwiesen, deren Eigenschaften als Komplexbildner auf das in den jeweiligen Komplexbildnern enthaltene Strukturelement des 2,2'-Bipyridin-, 1,10-Phenanthrolin- und 2,2',6',2''-Terpyridin-Ringsystems zurückgeführt werden kann, wie es beispielsweise bei den oben genannten Verbindungen der Formeln I bis IX der Fall ist.

Die Carboxylgruppen-tragenden Komplexbildher können aus den 1- bis 6-fach, vorzugsweise 1- bis 4-fach und besonders bevorzugt 1- bis 3-fach Methyl-substituierten Verbindungen I bis IX durch Oxidation der Methylgruppen, beispielsweise mittels Kaliumpermanganat, hergestellt werden. Die mit Methylgruppen substituierten oder benzanellierten Verbindungen I bis IX können beispielsweise nach den Verfahren, wie sie von Kröhnke et al., Synthesis 1 (1976); Sasse et al., J. Chem. Soc. 616 (1956); Sasse et al., J. Heterocycl. Chem. 8, 483 (1971); Bos et al., Synth. Commun. 9, 497 (1979) oder Elliot et al., J. Am. Chem. Soc. 104, 7519 (1982) beschrieben werden, erhalten werden. Die Oxidation der Methylgruppen zu Carboxylgruppen kann z.B. mit Kaliumpermanganat nach den allgemein anwendbaren Methoden wie sie von Anderson et al. (J. Chem. Soc. Dalton Trans. 2247 (1985)) oder Hanabusa et al. (Makromol. Chem. 190, 1 (1989)) beschrieben werden, durchgeführt werden. Von den Carboxylgruppen-tragenden, stickstoffhaltigen Komplexbildnern ist die Verwendung von 2,2'-Bipyridin-4,4'-dicarbonsäure und der 2,2'-Bipyridin-5,5'-dicarbonsäure bzw. von deren Salzen bevorzugt.

Bei der Durchführung des erfindungsgemäßen Verfahrens wird der Hydroformylierungsaustrag aus der Hydroformylierung mit "nacktem Rhodium", der außer dem Produktaldehyd unter anderem noch nicht abreagiertes Olefin sowie Produkte aus der Hydrierung des Produktaldehyds, beispielsweise die diesem entsprechenden Alkohole, gegebenenfalls ein Lösungsmittel, wie aromatische oder aliphatische Kohlenwasserstoffe oder höhermolekulare Kondensationsprodukte der im Zuge der Hydroformylierung gebildeten Aldehyde und den im Hydroformylierungsaustrag homogen gelösten Rhodium-Katalysator enthält, mit der wäßrigen Lösung des jeweils eingesetzten Komplexbildners versetzt, wobei sich ein wäßrigorganisches Zweiphasensystem ausbildet und wobei das in der organischen Phase gelöste Rhodium vom eingesetzten Komplexbildner nach Komplexbildung in die wäßrige Phase extrahiert wird. Der Zusatz eines Phasentransferkatalysators ist dabei nicht erforderlich, aber möglich.

Die Hydroformylierung kann in An- oder Abwesenheit von organischen Lösungsmitteln durchgeführt werden. Besonders vorteilhaft ist die Verwendung organischer Lösungsmittel, insbesondere bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende aromatische und aliphatische Kohlenwasserstoffe oder auch hochsiedende Aldehyd-Kondensationsprodukte, die im Zuge der Hydroformylierungsreaktion als Nebenprodukt als Folge der Kondensation der Produktaldehyde entstehen.

Zweckmäßigerweise wird der Hydroformylierungsaustrag vor seiner Extraktion auf Atmosphärendruck entspannt, obgleich es möglich ist, die Extraktion auch unter erhöhtem Druck oder unter vermindertem Druck vorzunehmen. Die Extraktion wird in der Regel bei Temperaturen von 20 bis 150°C, vorzugsweise bei 50 bis 120°C durchgeführt.

Um eine möglichst vollständige Extraktion des Rhodiums aus der organischen Phase des Hydroformylierungsaustrags zu erzielen, wird ein Molverhältnis Rhodium/Komplexbildner von mindestens 1/2 eingestellt, vorzugsweise wird jedoch ein Molverhältnis von 1/10 bis 1/200 gewählt.

Die Extraktion des Rhodiums aus der organischen Phase kann sowohl an der Luft als auch unter einer Schutzgasatmosphäre erfolgen. Die Anwendung einer Schutzgasatmosphäre, beispielsweise einer Stickstoff- oder Argonatmosphäre, ist insbesondere beim Vorliegen oxidationsempfindlicher Hydroformylierungsprodukte vorteilhaft. Zum Zwecke einer möglichst effizienten Extraktion ist eine gute Durchmischung der organischen und wäßrigen Phase erforderlich, wie sie bei Verwendung an sich herkömmlicher Extraktionsapparate, wie Mixer-Settler-Apparaturen, Blasensäulen und Gegenstromextraktionsapparaturen erzeugt werden kann. Der Übertritt des Rhodiums in die wäßrige Phase kann anhand von deren Rotfärbung gewünschtenfalls photometrisch verfolgt werden.

Die vom Rhodium-Katalysator befreiten Hydroformylierungsausträge können auf an sich übliche Weise aufgearbeitet werden. Der wäßrige, rhodiumhaltige Extrakt wird vorteilhaft in die Hydroformylierungsstufe zurückgeführt. Unter den dort üblicherweise herrschenden Reaktionsbedingungen - CO/H₂-Molverhältnis von 1/5 bis 5/1, vorzugsweise von 1/2 bis 2/1, Gesamtdruck von 10 bis 1000 bar, vorzugsweise von 90 bis 350 bar, Temperatur von 50 bis 170°C, insbesondere von 80 bis 140°C, wird das an den eingesetzten Komplexbildner koordinativ gebundene Rhodium durch das Kohlenmonoxid aus dem wasserlöslichen Komplex herausgelöst und der "nackte", lipophile Rhodium-Katalysator zurückgebildet und in die organische Phase des Hydroformylierungsmediums extrahiert, wohingegen der nunmehr rhodiumfreie, hydrophile Komplexbildner in der wäßrigen Phase verbleibt. Zur möglichst effizienten Ausnutzung dieses Effektes ist eine gute Durchmischung der organischen Phase des Hydroformylierungsmediums mit der wäßrigen Lösung des Rhodium-Komplexbiloner-Komplexes erforderlich, weshalb die Hydroformylierung vorteilhaft z.B in Rührautoklaven oder in mit strömungstechnischen Vorrichtungen, wie Umlenkblechen, versehenen Rohrreaktoren ausgeführt werden kann.

Das Volumenverhältnis des Volumens des Hydroformylierungsmediums zum Volumen der wäßrigen Rhodiumkomplex-Lösung ist im Prinzip nicht kritisch, man ist allerdings aus rein wirtschaftlichen Gründen bestrebt, den Volumenanteil der wäßrigen Phase am Gesamtvolumen von wäßriger und organischer Phase im Hydroformylierungsreaktor möglichst niedrig zu halten. Im allgemeinen wird ein Volumenverhältnis organische Phase/wäßrige Phase von 95/5 bis 30/70, insbesondere von 90/10 bis 50/50 bei der Hydroformylierung angewandt.

Nach beendeter Hydroformylierung kann das im Hydroformylierungsaustrag enthaltene Rhodium nach Erniedrigung des Kohlenmonoxid-Partialdrucks auf Werte von 0 bis 5 bar, vorzugsweise auf Werte von 0 bis 2 bar, besonders bevorzugt in Abwesenheit von Kohlenmonoxid, wieder vom Komplexbildher in die wäßrige Phase reextrahiert werden, und der Rhodium-Kreislauf kann aufs neue beginnen. Das erfindungsgemäße Verfahren kann somit sowohl im chargenweisen Betrieb als auch kontinuierlich durchgeführt werden.

Das erfindungsgemäße Verfahren eignet sich besonders gut zur Hydroformylierung von Olefinen mit mehr als 7 Kohlenstoffatomen, insbesondere zur Hydroformylierung von C₇- bis C₂₀-Olefinen, die geradkettig oder verzweigt sein können und die α-olefinische und/oder interne Doppelbindungen enthalten können, z.B. Octen-1, Dodecen-1 oder Trimer- und Tetramerpropylen. Die aus diesen Olefinen gebildeten Aldehyde sind Vorstufen für die Herstellung von Weichmacheralkoholen, die daraus auf herkömmliche Weise durch Hydrierung erzeugt werden können. Besonders gut geeignet ist das erfindungsgemäße Verfahren auch für die Hydroformylierung von polymeren Olefinen, wie niedermolekularem Polyisobuten. Das Hydroformylierungsprodukt des Polyisobutens wird durch reduktive Aminierung gemäß EP-A 244 616 in Polyisobutenamin umgewandelt, das als Kraftstoffadditiv dient. Die zur Hydroformylierung eingesetzten Olefine können z.B. durch die säurekatalysierte Wasserabspaltung aus den entsprechenden Fettalkoholen oder nach einer Vielzahl anderer technischer Verfahren, wie sie beispielsweise in Weissermel, Arpe: Industrielle Organische Chemie, 2. Aufl., S. 72 - 77 und S. 80 - 85, Verlag Chemie, Weinheim 1978 beschrieben sind, erzeugt werden und niedermolekulares Polyisobuten kann nach dem Verfahren von EP-A 145 235 produziert werden.

### Beispiele

### Beispiel 1

Dodecen-1 wurde in Gegenwart von "nacktem Rhodium", bei einem Rhodiumgehalt des Hydroformylierungsmediums von 100 ppm mit Synthesegas (CO/H₂-Molverhältnis: 1/1) bei einer Temperatur von 100°C und einem Druck von 100 bar zu den entsprechenden Tridecanalen umgesetzt. Der "nackte Rhodium-Katalysator" war im Hydroformylierungsmedium unter den Hydroformylierungsbedingungen aus Rhodiumdicarbonylacetonylacetonat erzeugt worden. 50 g des Hydroformylierungsaustrags, dessen Rhodiumgehalt infolge der durch die Umsetzung verursachten Massenzunahme auf 87 ppm Rhodium abgesunken war, wurden bei Atmosphärendruck zu einer Lösung von 0,11 g Natrium-2,2'-bipyridin-5-sulfonat in 50 g Wasser gegeben und eine halbe Stunde bei Raumtemperatur gerührt. Nach dieser Zeit enthielt die organische Phase 70 ppm Rhodium, die wäßrige Phase 20 ppm. Das Zweiphasen-Gemisch wurde unter Rühren auf 100°C erhitzt und noch eine weitere Stunde bei dieser Temperatur gerührt. Nach dieser Prozedur enthielt die organische Phase weniger als 5 ppm Rhodium, der Rhodiumgehalt der wäßrigen Phase war auf 85 ppm Rhodium, entsprechend einem Extraktionsgrad von mehr als 95 %, angestiegen.

### Beispiel 2

49,8 g einer wäßrigen rhodiumhaltigen Extraktlösung, wie sie gemäß dem Verfahren von Beispiel 1 erhalten worden war, wurde mit dem gleichen Volumen Trimerpropylen bei einer Temperatur von 100°C und Synthesegas (CO/H₂-Molverhältnis: 1/1) bei einem Druck von 150 bar 3 Stunden lang zur Reaktion gebracht. Danach war das Olefin bei einem Umsatz von 95 % mit einer Selektivität von 98 % in die entsprechenden Aldehyde umgewandelt. Der zweiphasige Reaktionsaustrag enthielt in der organischen Phase 9,8 mg Rhodium und in der wäßrigen Phase 1,3 mg Rhodium. Die beiden Phasen wurden bei Atmosphärendruck und bei einer Temperatur von 85°C eine Stunde lang intensiv durchmischt. Anschließend befanden sich in der organischen Phase noch 1,8 mg Rhodium, der Rhodiumgehalt der wäßrigen Phase betrug 9,0 mg Rhodium.

Die wäßrige Phase wurde in den Hydroformylierungsreaktor zurückgeleitet, dort mit dem gleichen Volumen an Trimerpropylen versetzt und das Olefin, wie oben beschrieben, hydroformyliert. Nach beendeter Hydroformylierung war das Trimerpropylen bei einem Umsatz von 94 % und mit einer Selektivität von 98 % in die entsprechenden Aldehyde umgesetzt. Der zweiphasige Reaktionsaustrag enthielt 7,5 mg Rhodium in der organischen und 1,5 mg Rhodium in der wäßrigen Phase. Nachdem die organische Phase, wie oben beschrieben, extrahiert worden war, betrug der Rhodiumgehalt der wäßrigen Phase 7,7 mg, der der organischen Phase 0,6 mg. Die wäßrige Rhodiumlösung wurde erneut, unter den angegebenen Bedingungen zur Hydroformylierung eingesetzt und die betreffenden Aldehyde bei einem Olefinumsatz von 94 % mit 98 % Selektivität erhalten. Die organische Phase des Hydroformylierungsaustrags enthielt 5,2 mg Rhodium, die wäßrige 0,53 mg Rhodium.

Die Rhodium-Verluste während dieses Reihenversuchs begründen sich durch Verluste bei Probenahmen zu Analysenzwecken.

### Beispiel 3

43,8 g einer nach dem Verfahren von Beispiel 1 hergestellten wäßrigen Rhodiumlösung wurden mit 50 g Octen-1 im Autoklaven bei einer Temperatur von 100°C und einem Druck von 150 bar mit Synthesegas 3 Stunden lang umgesetzt. Nach beendeter Reaktion enthielt der Hydroformylierungsaustrag 1,24 mg Rhodium, die wäßrige Phase 1,43 mg Rhodium. Nach der Extraktion gemäß dem Verfahren von Beispiel 2 war der Rhodiumgehalt der organischen Phase auf 0,25 abgesunken, der der wäßrigen Phase auf 2,53 mg angestiegen. Der Umsatz bei der Hydroformylierung betrug 99 % bei einer Aldehydselektivität von 95 %. Die wäßrige Phase wurde erneut zur Hydroformylierung eingesetzt, wobei praktisch das gleiche Ergebnis, wie zuvor angegeben, erzielt wurde.

### Beispiel 4

37,7 g Polyisobuten (mittleres Molekulargewicht ca. 1000) und 40 g Toluol wurden mit 41,1 g einer wäßrigen, nach dem Verfahren von Beispiel 1 hergestellten Rhodiumlösung, versetzt und bei einer Temperatur von 130°C und einem Druck von 280 bar CO/H₂ drei Stunden zur Reaktion gebracht. Nach der Rhodiumextraktion wurde die organische Phase abgetrennt und analysiert.

Ausbeute: 93 % Hydroformylierungsprodukt

### Beispiel 5

100 g Trimerpropylen wurden in Gegenwart von 100 ppm "nacktem Rhodium", das gemäß Beispiel 1 im Hydroformylierungsmedium erzeugt worden war, bei einer Temperatur von 130°C und einem Synthesegas-Druck von 280 bar fünf Stunden lang umgesetzt. 50 g des Hydroformylierungsaustrags, enthaltend 5 mg (0,049 Mol Rhodium) wurden mit einer Lösung von 602,1 mg (0,49 Mol) Tetranatrium-meso-tetra(4-sulfophenyl)porphin-dodecahydrat (hergestellt nach: J. Org. Chem. 38, 2103 (1973)) in 50 ml Wasser versetzt und drei Stunden bei 85°C an der Luft gerührt. Anschließend befanden sich in der wäßrigen Phase 95 ppm Rhodium, in der organischen Phase waren 3 ppm Rhodium verblieben, entsprechend einem Extraktionsgrad von 97 %.

### Beispiel 6

50 g eines 70 ppm "nacktes Rhodium" enthaltenden Austrags aus der Hydroformylierung von Trimerpropylen wurde bei einer Temperatur von 85°C mit 50 g einer wäßrigen Lösung von 135 mg Natrium-1,10-phenanthrolin-sulfonat · 1,5 H₂O intensiv gerührt. Nach 2 h enthielt die wäßrige Phase 65 ppm Rhodium, entsprechend einem Extraktionsgrad von 93 %.

Natrium-1,10-phenanthrolin-sulfonat · 1,5 H₂O war analog dem Verfahren zur Sulfonierung von 2,2'-Bipyridin ausgehend von 1,10-Phenanthrolin hergestellt worden (vgl. Chem.Ber. 123, 1953 (1990)).

Ein anderes Verfahren zur Sulfonierung von 1,10-Phenanthrolin ist in Anal. Chem. 33, 867 (1961) angegeben.

### Beispiel 7

1484 g Trimerpropylen und 36,1 mg Rh(CO)₂acac wurden in einen Autoklaven eingefüllt, 100 bar CO/H₂ im Molverhältnis 1/1 aufgepreßt, entspannt und nochmals aufgepreßt, auf 130°C aufgeheizt und CO/H₂ bis zu einem Druck von 280 bar nachgepreßt. Nach drei Stunden wurde abgekühlt, entspannt und die Reaktionsmischung ausgetragen. Nach Filtration enthielt der Hydroformylierungsaustrag 74 ppm Rhodium. 120 g dieses Austrages wurden unter Argon mit 319 mg Dinatrium-2,2'-bipyridin-4,4'-dicarboxylat-hydrat (0,9 mmol) in 120 ml Wasser vermischt, auf 85°C erhitzt und dabei kräftig gerührt. Nach 90 min enthielt die wäßrige Phase 71, die organische 7 ppm des Rhodiums, was einem Extraktionsgrad von 92,92 % entspricht. acac: Acetonylacetonat

## Patentansprüche

1. Verfahren zur Herstellung von Aldehyden durch die Hydroformylierung von Olefinen mit mehr als 7 Kohlenstoffatomen, umfassend die Stufe der Hydroformylierung mittels eines homogen im Reaktionsmedium gelösten nackten Rhodium-Katalysators, die Abtrennung des nackten Rhodium-Katalysators vom Austrag der Hydroformylierungsreaktion und die Rückführung des vom Hydroformylierungsaustrag abgetrennten Rhodiums in die Hydroformylierungsstufe, dadurch gekennzeichnet, daß man den nackten Rhodium-Katalysator aus dem Hydroformylierungsaustrag mittels einer wäßrigen Lösung eines stickstoffhaltigen Komplexbildners aus der Gruppe der gegebenenfalls substituierten, sulfonierten oder sulfonierte Substituenten tragenden 2,2'-Bipyridine, 1,10-Phenanthroline, 2,2'-Bichinoline, 2,2',6',2''-Terpyridine und Porphine, und/oder aus der Gruppe der gegebenenfalls substituierten, carboxylierten oder carboxylierte Substituenten tragenden 2,2'-Bipyridine, 1,10-Phenanthroline, 2,2'-Bichinoline, 2,2',6',2''-Terpyridine und Porphine, in die wäßrige Phase extrahiert und den wäßrigen, rhodiumhaltigen Extrakt in die Hydroformylierungsstufe zurückführt.

2. Verfahren nach Anspruch 1, dadurch gekennzeichnet, daß man als Komplexbildner ein 2,2'-Bipyridin-Sulfonat, ein 1,10-Phenanthrolin-Sulfonat, ein 2,2'-Bichinolin-Sulfonat oder ein 2,2',6',2''-Terpyridin-Sulfonat verwendet.

3. Verfahren nach Anspruch 1, dadurch gekennzeichnet, daß man als Komplexbildner ein wasserlösliches Salz der 2,2'-Bipyridin-5-Sulfonsäure verwendet.

4. Verfahren nach Anspruch 1, dadurch gekennzeichnet, daß man als Komplexbildner ein 2,2'-Bipyridin-dicarboxylat, ein 1,10-Phenanthrolin-dicarboxylat, ein 2,2'-Bichinolin-dicarboxylat oder ein 2,2',6',2''-Terpyridin-dicarboxylat verwendet.

5. Verfahren nach Anspruch 1, dadurch gekennzeichnet, daß man als Komplexbildner ein wasserlösliches Salz der 2,2'-Bipyridin-4,4'-dicarbonsäure und/oder der 2,2'-Bipyridin-5,5'-dicarbonsäure verwendet.

## Claims

1. A process for the preparation of an aldehyde by the hydroformylation of an olefin of more than 7 carbon atoms, comprising the stage of hydroformylation by means of a bare rhodium catalyst homogeneously dissolved in the reaction medium, separation of the bare rhodium catalyst from the mixture discharged from the hydroformylation reaction and recycling of the rhodium separated from said mixture to the hydroformylation stage, wherein the bare rhodium catalyst is extracted from the reacted hydroformylation mixture into the aqueous phase by means of an aqueous solution of a nitrogen-containing complexing agent selected from the group consisting of the unsubstituted or substituted 2,2'-bipyridines, 1,10-phenanthrolines, 2,2'-biquinolines, 2,2',6',2''-terpyridines and porphines which are sulfonated or carry sulfonated substituents, and/or from the group consisting of the unsubstituted or substituted 2,2'-bipyridines, 1,10-phenanthrolines, 2,2'-biquinolines, 2,2',6',2''-terpyridines and porphines which are carboxylated or carry carboxylated substituents, and the aqueous, rhodium-containing extract is recycled to the hydroformylation stage.

2. A process as claimed in claim 1, wherein a 2,2'-bipyridinesulfonate, a 1,10-phenanthrolinesulfonate, a 2,2'-biquinolinesulfonate or a 2,2',6',2''-terpyridinesulfonate is used as the complexing agent.

3. A process as claimed in claim 1, wherein a water-soluble salt of 2,2'-bipyridine-5-sulfonic acid is used as the complexing agent.

4. A process as claimed in calim 1, wherein a 2,2'-bipyridinedicarboxylate, a 1,10-phenanthrolinedicarboxylate, a 2,2'-biquinolinedicarboxylate or a 2,2',6',2''-terpyridinedicarboxylate is used as the complexing agent.

5. A process as claimed in claim 1, wherein a water-soluble salt of 2,2'-bipyridine-4,4'-dicarboxylic acid and/or of 2,2'-bipyridine-5,5'-dicarboxylic acid is used as the complexing agent.

## Revendications

1. Procédé de préparation d'aldéhydes par l'hydroformylation d'oléfines a plus de 7 atomes de carbone, comprenant l'étape d'hydroformylation au moyen d'un catalyseur au rhodium nu dissous dans le milieu réactionnel, la séparation du catalyseur au rhodium nu d'avec le produit de la réaction d'hydroformylation et le renvoi du rhodium séparé du produit de l'hydroformylation dans l'étape d'hydroformylation, caractérisé en ce que l'on extrait en phase aqueuse le catalyseur au rhodium nu a partir du produit d'hydroformylation au moyen d'une solution aqueuse d'un complexant azoté du groupe des 2,2'-bipyridines, des 1,10-phénanthrolines, des 2,2'-biquinoléines, des 2,2',6',2''-terpyridines et des porphines éventuellement substituées, sulfonées ou portant des substituants sulfonés, et/ou du groupe des 2,2'-bipyridines, des 1,10-phénanthrolines, des 2,2'-biquinoléines, des 2,2',6',2''-terpyridines et des porphines éventuellement substituées, carboxylées ou portant des substituants carboxylés, et on renvoie dans l'étape d'hydroformylation l'extrait aqueux contenant du rhodium.

2. Procédé selon la revendication 1, caractérisé en ce que l'on utilise, comme complexant, un 2,2'-bipyridinesulfonate, un 1,10-phénanthrolinesulfonate, un 2,2'-biquinoléinesulfonate ou un 2,2',6',2''-terpyridinesulfonate.

3. Procédé selon la revendication 1, caractérisé en ce que l'on utilise, comme complexant, un sel hydrosoluble de l'acide 2,2'-bipyridine-5-sulfonique.

4. Procédé selon la revendication 1, caractérisé en ce que l'on utilise, comme complexant, un 2,2'-bipyridinedicarboxylate, un 1,10-phénanthrolinedicarboxylate, un 2,2'-biquinoléinedicarboxylate ou un 2,2',6',2''-terpyridinedicprboxylate.

5. Procédé selon la revendication 1, caractérisé en ce que l'on utilise, comme complexant, un sel hydrosoluble de l'acide 2,2'-bipyridine-4,4'-dicarboxylique et/ou de l'acide 2,2'-bipyridine-5,5'-dicarboxylique.
